(19) Europäisches Patentamt
European Patent Office
Office européen des brevets

(11) Numéro de publication: **0 382 635 B1**

# (12) FASCICULE DE BREVET EUROPEEN

(45) Date de publication de fascicule du brevet: **30.11.94**

(51) Int. Cl.5: **A61K 31/50**, A61K 31/535, C07D 237/20

(21) Numéro de dépôt: **90400335.7**

(22) Date de dépôt: **07.02.90**

## (54) Utilisation de dérivés d'alkyl-5 pyridazine comme médicaments actifs sur le système cholinergique.

(30) Priorité: **07.02.89 FR 8901546**

(43) Date de publication de la demande:
**16.08.90 Bulletin 90/33**

(45) Mention de la délivrance du brevet:
**30.11.94 Bulletin 94/48**

(84) Etats contractants désignés:
**AT BE CH DE DK FR GB IT LI LU NL SE**

(56) Documents cités:
**EP-A- 0 382 634**
**FR-A- 2 510 998**

**J. MED. CHEM., vol. 32, mars 1989, pages 528-537, American Chemical Society; C.-G. WERMUTH et al.: "3-Aminopyridazine derivatives with atypical antidepressant, serotonergic, and dopaminergic activities"**

(73) Titulaire: **SANOFI**
**32-34, rue Marbeuf**
**F-75008 Paris (FR)**

(72) Inventeur: **Boigegrain, Robert**
**Lou Pradas N 20 Jacou**
**F-34830 Clapiers (FR)**
Inventeur: **Wermuth, Camille Georges**
**3, rue de la Côte d'Azur**
**F-67100 Strasbourg (FR)**
Inventeur: **Worms, Paul**
**10, rue du Devois**
**F-34980 St Gely Du Fesc (FR)**

(74) Mandataire: **Gillard, Marie-Louise et al**
**Cabinet Beau de Loménie**
**158, rue de l'Université**
**F-75340 Paris Cédex 07 (FR)**

EP 0 382 635 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

Depuis de nombreuses années, des dérivés de pyridazine ont été proposés en tant que médicaments.

Dans un grand nombre de cas, il s'agit de substances actives sur le système cardiovasculaire, présentant en particulier un effet hypotenseur ou vasodilateur; dans d'autres cas on a mentionné pour des dérivés de pyridazine une action antiinflammatoire et analgésique.

Enfin les brevets français 2 141 697, 2 510 997 et 2 510 998 décrivent des dérivés de pyridazine diversement substitués sur le cycle pyridazinique et portant tous en position 3 un substituant aminé du type

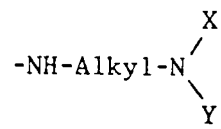

où X et Y représentent indépendamment l'hydrogène, un groupe alkyle ou forment avec l'atome d'azote auquel ils sont liés un hétérocycle,tel que la morpholine.

Tous ces composés présentent une activité sur le système nerveux central en tant qu'antidépresseurs.

Plus particulièrement le brevet français n° 2 510 998 décrit une famille de dérivés de pyridazine comprenant parmi ses membres un dérivé alkyl-5 pyridazine,à savoir la (morpholino-2 éthylamino)-3 méthyl-5 phényl-6 pyridazine (I).

Ce composé présente des propriétés antidépressives d'intensité moyenne. Des études ultérieures ont montré qu'il possède également d'intéressantes propriétés en tant que ligand des récepteurs cholinergiques.

Chez les mammiféres, il existe deux sous classes de récepteurs cholinergiques muscariniques : les récepteurs $M_1$ et $M_2$

Les récepteurs de type $M_1$ sont concentrés dans certaines zones du cerveau telles que l'hippocampe, le cortex cérébral, le striatum ainsi que dans les ganglions sympathiques. Ces sites de liaison peuvent être sélectivement marqués par la [$^3$H] pirenzépine. Ceux de type $M_2$ prédominent dans le coeur et dans l'iléon et peuvent être marqués par le [$^3$H] N-méthylscopolamine.

Les démences séniles et notamment les démences de type Alzheimer sont des affections graves dont la fréquence tend à augmenter en fonction de l'augmentation de la longévité de la population.

Les études entreprises par différents auteurs, ont mis en évidence dans ces maladies l'existence d'un déficit spécifique des marqueurs cholinergiques corticaux provoquant des troubles graves des fonctions supérieures.

Les résultats obtenus par utilisation d'agonistes muscariniques pour le traitement des démences séniles se sont montrés encourageants. Toutefois les agonistes muscariniques n'existent qu'en petit nombre et se sont avérés de maniement difficile chez l'homme.

Par suite la recherche d'agonistes muscariniques post-synaptiques comme traitement des démences séniles s'avère aujourd'hui tout à fait souhaitable.

L'intérêt de disposer d'agonistes muscariniques central sélectifs pour remédier au déficit cholinergique dans la maladie d'Alzheimer a été mentionné notamment dans ISI Atlas of Science : Pharmacology (1987), p. 98 à 100.

La présente invention a donc pour objet l'utilisation de dérivés d'alkyl-5 pyridazine de formule :

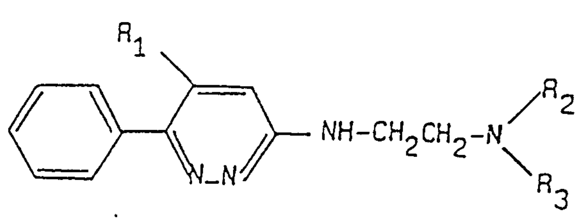

(II)

où $R_1$ représente un groupe alkyle en $C_1$-$C_4$ ou un groupe phényle ; $R_2$ et $R_3$ représentent indépendamment un groupe alkyle en $C_1$-$C_4$ ou encore $R_2$ et $R_3$ considérés avec l'atome d'azote auquel ils sont liés, constituent un groupe morpholino,
ou d'un de leurs sels pharmaceutiquement acceptables pour la préparation de compositions pharmaceutiques pour le traitement des syndromes dégénératifs liés à la sénescence causés par un déficit cholinergique central, et notamment pour le traitement des démences séniles.

Les composés de formule (II)dans laquelle $R_1$ est un groupe alkyle en $C_2$-$C_4$, et $R_2$ et $R_3$ sont tels que définis ci-dessus sont nouveaux et constituent un autre aspect de l'invention, ainsi que les sels que ces composés sont susceptibles de former avec les acides pharmaceutiquement acceptables.

Parmi ces composés ceux où $R_1$ est $-CH_2CH_3$ ou $-CH_2CH_2CH_3$ sont particulièrement préférés.

Les composés de formule (II) peuvent être préparés par action d'une amine

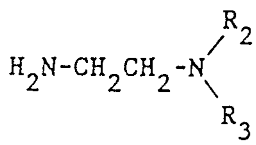
(1)

sur le dérivé chloré

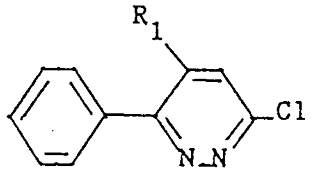
(2)

La réaction est effectuée par chauffage entre 100 et 150 °C du dérivé chloré (2° avec un large excès d'amine (1) éventuellement en présence de chlorure d'ammonium.

On opère sans solvant ou dans un solvant inerte comme le n-butanol.

Les dérivés chlorés (2) peuvent être préparés à partir d'une phénylcétone (3) suivant le schéma réactionnel:

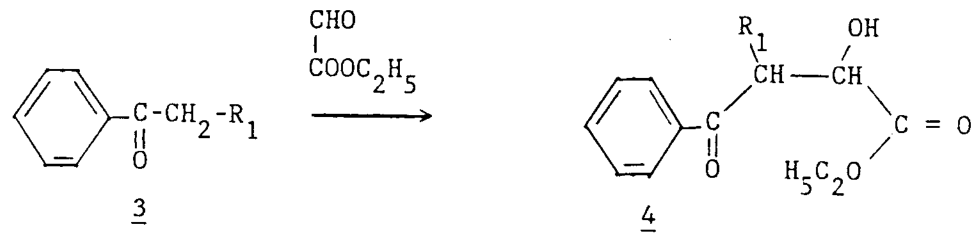


3 4

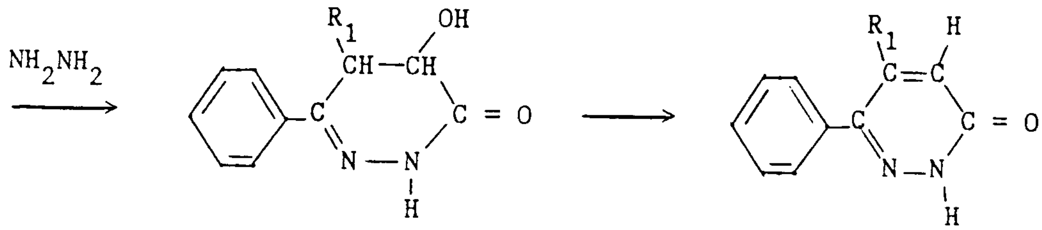


5 6

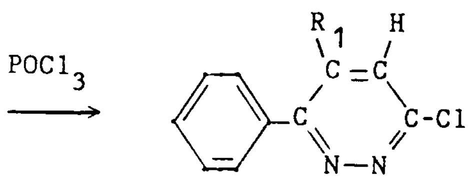


2

A partir de la cétone 3, par chauffage avec le glyoxylate d'éthyle à une température comprise entre 80 et 140 °C, on obtient l'hydroxy céto ester 4. Le mélange réactionnel brut est alors repris dans un solvant inerte tel que le n-butanol et additionné d'hydrate d'hydrazine. Par chauffage au reflux pendant 24 heures, on obtient l'hydroxypyridazinone 5 qui chauffée en milieu acide conduit par déshydratation à la 2H-pyridazinone-3 6.

Cette dernière chauffée avec de l'oxychlorure de phosphore en excès fournit la chloro-3 pyridazine 2. On opère sans solvant ou en présence d'un solvant inerte tel que l'acétonitrile.

Dans tous les cas, les produits (II) isolés sous forme de base peuvent être salifiés par action d'une quantité équimoléculaire d'un acide pharmaceutiquement acceptable au sein d'un solvant convenable.

Les exemples suivants illustrent la préparation des composés de formule (II)

EXEMPLE 1

(Morpholino-2 éthylamino)-3 méthyl-5 phényl-6 pyridazine dioxalate (CM 30316).

(II) $R_1 = CH_3$ 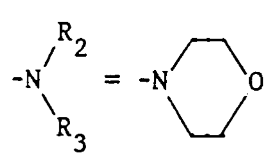

A) Chloro-3 méthyl-5 phényl-6 pyridazine.

1. Hydroxy-2 méthyl-3 phényl-4 oxo-4 butyrate d'éthyle.

On chauffe à 135 °C pendant 5 heures, le mélange de 13,4 g de propiophénone et 15,3 g de glyoxylate d'éthyle.

Le produit ainsi obtenu est utilisé tel quel pour l'opération suivante.

2. Méthyl-5 phényl-6 2H-pyridazinone-3.

On dissout le produit brut obtenu ci-dessus dans 150 ml de n-butanol, puis on ajoute 9,44 ml d'hydrate d'hydrazine et chauffe au reflux pendant 24 heures.

On distille à pression ordinaire une partie du n-butanol de façon à éliminer l'eau formée dans la réaction sous forme d'azéotrope. On concentre ensuite à siccité sous vide. On reprend le résidu dans un mélange de 100 ml d'acide acétique et 10 ml d'acide chlorhydrique concentré. On chauffe le mélange à 100 °C pendant 4 heures. On verse la solution dans l'eau froide et laisse cristalliser. On essore le solide et sèche.

Poids : 11,6 g F : 218 °C.

3. Chloro-3 méthyl-5 phényl-6 pyridazine.

A 12 g de pyridazinone obtenue ci-dessus on ajoute 50 ml d'oxychlorure de phosphore et chauffe à 80 °C pendant 4 heures.

On verse lentement le mélange sur de la glace et alcalinise avec une solution de soude à 20 %.

On essore le précipité, lave abondamment avec de l'eau et recristallise dans l'isopropanol. On obtient 9,9 g du produit attendu.

F : 122 °C.

B) CM 30316

On chauffe à reflux pendant 12 heures un mélange de 8 g du dérivé chloré obtenu ci-dessus et 10 g de morpholino-2 éthylamine dans 80 ml de n-butanol.

On verse la solution chaude dans 200 ml d'eau et filtre le précipité qu'on lave avec un peu d'éther. On sépare la phase aqueuse que l'on extrait avec de l'éther. On réunit les extraits éthérés et on les extrait avec une solution d'acide sulfurique 1N.

On sépare la phase aqueuse acide et alcalinise avec une solution de carbonate de sodium à 10 %. On extrait avec de l'acétate d'éthyle, sèche la solution sur sulfate de sodium et évapore à siccité sous vide.

On dissout la base obtenue dans l'éther isopropylique et ajoute 2 équivalents d'acide oxalique. On chauffe à l'ébullition jusqu'à dissolution. Par refroidissement le dioxalate cristallise. On essore et recristallise dans le même solvant.

Poids : 6 g F : 182-183 °C.

EXEMPLES 2 à 7

A) En opérant comme dans l'exemple 1A mais en faisant varier la cétone de départ, on obtient les chloro-3 pyridazines réunies dans le tableau suivant :

TABLEAU 1

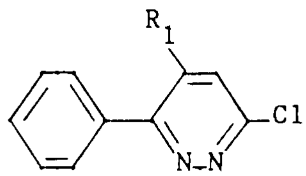

| $R_1$ | Constantes physiques |
|---|---|
| $-CH_2CH_3$ | F : 70°C |
| $-CH_2CH_2CH_3$ | F : 60°C |
| $-C_6H_5$ | - |

A partir de ces dérivés chlorés et de celui de l'exemple 1A en faisant varier les amines utilisées, on obtient suivant la technique de l'exemple 1B, les composés (II) rassemblés dans le tableau 2.

TABLEAU 2

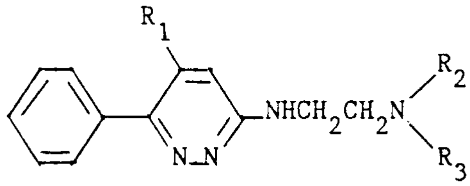

| Ex. | N° Réf. | $R_1$ | 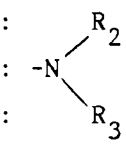 | Sel F : °C |
|---|---|---|---|---|
| 2 | SR96169 A. | $-CH_2CH_3$ | 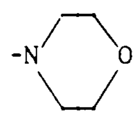 | Monooxalate 1,5$H_2O$ : 195 |
| 3 | SR96186 A: | $-CH_2CH_2CH_3$ | " " | Monooxalate : 195 |
| 4 | SR96150 A: | $-CH_3$ | 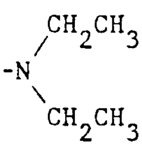 | Dioxalate : 152 |
| 5 | SR96180 A: | $-CH_2CH_3$ | " " | Dioxalate : 106 |
| 6 | SR96187 A: | $-CH_2CH_2CH_3$ | " " | Dioxalate : 143 |
| 7 | CM30365 | $-C_6H_5$ | 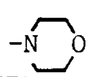 | Dichlorhydrate: 235°C |

Les composés de formule (II) ont été étudiés en ce qui concerne leurs propriétés thérapeutiques et notamment en ce qui concerne leur affinité pour les récepteurs cholinergiques muscariniques.

In vitro nous avons étudié l'intéraction des produits (II) avec les fixations à haute affinité de la pirenzépine tritiée et de la N-méthylscopolamine tritiée sur des membranes d'hippocampe de rat et de muscle lisse d'iléon de cobaye, respectivement.

Methodologies

A) Recherche d'une affinité pour le récepteur cholinergique muscarinique de type $M_1$ .

L'intéraction des molécules avec les récepteurs muscariniques de type $M_1$ a été étudiée en mesurant in vitro sur homogénat d'hippocampe de rat, le déplacement de la pirenzépine tritié ([$^3$H] PZ) de ses sites de fixation spécifiques. Des aliquots (100 $\mu$l) d'un homogénat d'hippocampe de rat à 5 % (P/v) dans un tampon $Na_2HPO_4$ (50 mM, pH 7,40), sont incubés 2 h à 4 °C en présence de [$^3$H] PZ (76 Ci/mmole ; 1 nM final) et de concentrations croissantes en produits à étudier. Le volume final est de 2 ml. La réaction est arrêtée par centrifugation 10 mn à 50.000 xg. Après décantation et lavage des culots, la radioactivité fixée est comptée par scintillation liquide. La fixation non spécifique est déterminée en présence de 10 $\mu$M de sulfate d'atropine. La concentration inhibitrice 50 ($CI_{50}$) est déterminée

graphiquement.

(Réf. : Watson J.D., Roeskoe W.R. and Yamamura H.I., Life Sci., 31, 2019-2029, 1982).

B) Recherche d'une affinité pour le récepteur cholinergique muscarinique de type $M_2$ .

L'intéraction avec les récepteurs muscariniques de type $M_2$ a été étudiée en mesurant in vitro sur un homogénat de muscle lisse d'iléon de cobaye, le déplacement de la N-méthyl-scopolamine tritiée ([$^3$H] NMS) de ses sites de fixation spécifiques. Des aliquots (50 µl) d'un homogénat de muscle lisse de cobaye à 0,625 % (P/v) dans du tampon HEPES (20 mM) contenant NaCl (100 mM) et $MgCl_2$ (10 mM) (pH final : 7,5), sont incubés 20 mn à 30 °C en présence de [$^3$H] NMS (85 Ci/mmole ; 0,3 nM final) et de concentrations croissantes en produits à tester. Le volume final est de 1 ml. La réaction est arrêtée par centrifugation 5 mn à 15.000 xg. La fixation non spécifique est déterminée en présence de 10 µM de sulfate d'atropine.

(Réf. : Hammer R., Berrie C.P., Birdsall N.I.M, Burgen A.S.V. and Hulme E.C., Nature, 283, 90-92, 1980.

Hulme E.C., Birdsall, N.I.M., Burgen A.S.V. and Mettha P., Mol. Pharmacol., 14, 737-750, 1978).

Resultats

Le tableau 3 indique les affinités des produits de l'invention pour les récepteurs $M_1$ et $M_2$ . Les résultats sont exprimés en concentrations inhibitrices 50 pour cent ($CI_{50}$), soit la concentration (en µM) qui inhibe de 50 % la fixation du ligand tritié aux récepteurs membranaires. La $CI_{50}$ de déplacement de la $^3$H-pirenzépine représente l'affinité pour le récepteur $M_1$ ; la $CI_{50}$ de déplacement de la $^3$H-NMS représente l'affinité pour le récepteur $M_2$.

De plus dans la 3e colonne on a indiqué le rapport r entre les les $CI_{50}$ $M_2$ et $M_1$ qui exprime la sélectivité des produits vis-à-vis du récepteur $M_1$.

TABLEAU 3

| Produit n° | 3H-PZ (M1) CI50 µM | 3H-NMS (M2) CI50 µM | r = M2/M1 |
|---|---|---|---|
| CM 30316 | 0,55 | 100 | 182 |
| SR 96169 A | 3,7 | 30 | 8 |
| SR 96186 A | 0,6 | 18 | 30 |
| SR 96150 A | 0,06 | 2,8 | 47 |
| SR 96180 A | 0,07 | 1 | 14 |
| SR 96187 A | 0,4 | 3,5 | 9 |

Ces résultats montrent que les composés selon l'invention présentent une forte affinité pour les récepteurs cholinergiques muscariniques avec une spécificité marquée pour les récepteurs centraux de type $M_1$.

Par ailleurs, les composés de formule (II) ont été soumis à une étude in vivo.

La pirenzépine (PZ) est un antagoniste spécifique des ligands des récepteurs cholinergiques muscariniques centraux $M_1$. L'injection intrastriatale de PZ chez la souris induit un comportement rotatoire. On a étudié l'antagonisme de ce comportement par les produits à étudier.

Les produits selon l'invention sont administrés par voie orale après avoir été solubilisés dans l'eau distillée ou mis en suspension dans une solution de gomme arabique à 5 %. Les contrôles sont réalisés par injection du solvant pur dans les mêmes conditions.

Les animaux utilisés sont des souris femelles (Swiss, CD 1, Charles River, France) de poids corporel entre 25 et 30 grammes.

La pirenzépine est mise en solution dans un tampon phosphate, le pH de la solution est 6.

Les produits à étudier ou leurs solvants sont administrés par voie orale sous un volume de 0,4 ml pour 20 g de poids corporel, 4 heures avant injection directe de pirenzépine à la dose de 1 µg de produit dans 1 µl de solvant dans le striatum droit de la souris, selon la méthode décrite par P. Worms et al. dans Psychopharmacology 1987, 93, 489-493.

Le nombre de rotations contralatérales (sens opposé au côté de l'injection) a été compté pendant trois périodes de 2 minutes après injection de pirenzépine : minutes 2 à 4, 8 à 10 et 13 à 15. Chaque traitement comporte 10 animaux par dose. Pour chaque traitement, on calcule le nombre total de rotations et le pourcentage d'antagonisme vis-à-vis du lot contrôle.

Dans le tableau 4 sont réunis les résultats obtenus avec les produits de formule (II) administrés par voie orale à la dose de 3 mg/kg de poids corporel.

TABLEAU 4

| Produits n° | % inhibition rotations PZ 3 mg/kg p.o. |
|---|---|
| CM 30316 | - 20 % * |
| SR 96169 A | - 46 % ** |
| SR 96186 A | - 41 % ** |
| SR 96150 A | - 49 % ** |
| SR 96180 A | - 38 % ** |
| SR 96187 A | - 37 % * |
| Test t de Student | |

* p < 0,05
** p < 0,01

Enfin les composés de formule (II) n'ont manifesté aucun signe de toxicité aux doses où ils sont actifs.

Par suite les composés (II) peuvent être utilisés en tant que médicaments dans tous les cas où se manifeste un déficit cholinergique cortical notamment pour le traitement des syndromes dégénératifs liés à la sénescence et particulièrement les troubles de la mémoire et les démences séniles.

En conséquence selon un autre de ses aspects, la présente demande concerne les compositions pharmaceutiques contenant au moins un des composés de formule (II) ou un de leurs sels en tant qu'ingrédient actif.

Dans les compositions pharmaceutiques de la présente invention pour l'administration orale, sublinguale, transdermique ou rectale, les ingrédients actifs de formule (II) ci-dessus peuvent être administrés sous forme unitaire d'administration, en mélange avec les supports pharmaceutiques classiques, aux êtres humains notamment pour le traitement des démences séniles. Les formes unitaires d'administration appropriées comprennent les formes par voie orale, telles que les comprimés, les gélules, les poudres, les granulés et les solutions ou suspensions orales, les formes d'administration sublinguale et buccale, les formes d'administration sous-cutanée, intramusculaire ou intraveineuse et les formes d'administration rectale.

Afin d'obtenir l'effet désiré, la dose de principe actif peut varier entre 20 et 500 mg par jour.

Chaque dose unitaire peut contenir de 5 à 200 mg d'ingrédient actif en combinaison avec un support pharmaceutique. Cette dose unitaire peut être administrée 1 à 4 fois par jour.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans les gélules molles ou dures.

Les poudres ou les granulés dispersibles dans l'eau peuvent contenir l'ingrédient actif en mélange avec des agents de dispersion ou des agents mouillants, ou des agents de mise en suspension comme la polyvinylpyrrolidone, de même qu'avec des édulcorants ou des correcteurs de goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol et le butylèneglycol.

Le principe actif peut être formulé également sous forme de microcapsules, éventuellement avec un ou plusieurs supports ou additifs.

A titre de préparation galénique on peut préparer des gélules contenant :

| | |
|---|---|
| Principe actif | 0,010 g |
| Lactose | 0,050 g |
| Stéarate de magnésium | 0,005 g |

en mélangeant intimement les ingrédients ci-dessus et en versant le mélange dans des gélules de gélatine dure.

## Revendications

1. Utilisation de dérivés d'alkyl-5 pyridazine de formule :

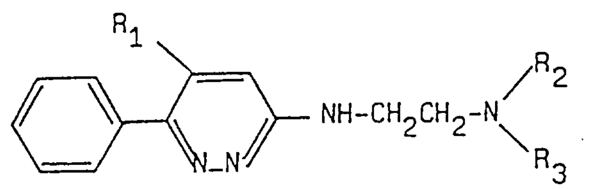

(II)

où $R_1$ représente un groupe alkyle en $C_1$-$C_4$ ou un groupe phényle ; $R_2$ et $R_3$ représentent indépendamment un groupe alkyle en $C_1$-$C_4$ ou encore $R_2$ et $R_3$ considérés avec l'atome d'azote auquel ils sont liés, constituent un groupe morpholino,
ou d'un de leurs sels pharmaceutiquement acceptables pour la préparation de compositions pharmaceutiques pour le traitement des syndromes dégénératifs liés à la sénescence causés par un déficit cholinergique central.

2. Utilisation selon la revendication 1, caractérisée en ce que les compositions pharmaceutiques sont utilisées pour le traitement des démences séniles.

3. Utilisation selon l'une des revendications 1 ou 2, caractérisée en ce que les compositions pharmaceutiques sont sous la forme de doses unitaires contenant de 5 à 200 mg du composé de formule (II) en mélange avec un excipient pharmaceutique.

4. Dérivés d'alkyl-5 pyridazine de formule :

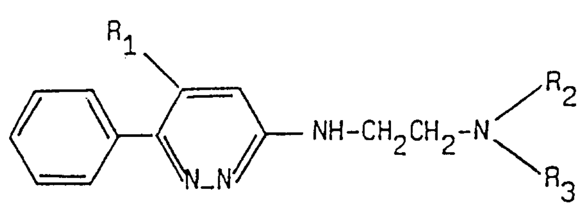

(II)

dans laquelle $R_1$ représente un groupe alkyle en $C_2$-$C_4$ ;
$R_2$ et $R_3$ représentent indépendamment un groupe alkyle en $C_1$-$C_4$ ou encore $R_2$ et $R_3$ considérés avec l'atome d'azote auquel ils sont liés constituent un groupe morpholino,
ou d'un de leurs sels pharmaceutiquement acceptables.

5. Dérivés selon la revendication 4, caractérisé en ce que $R_1$ est $-CH_2-CH_3$ ou $-CH_2-CH_2-CH_3$.

6. Procédé pour l'obtention d'un dérivé d'alkyl-5 pyridazine selon l'une des revendications 4 ou 5, caractérisé en ce qu'il consiste à faire réagir une amine de formule

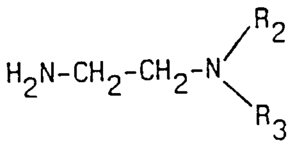

(1)

dans laquelle $R_2$ et $R_3$ sont tels que définis ci-dessus avec un dérivé chloré de formule :

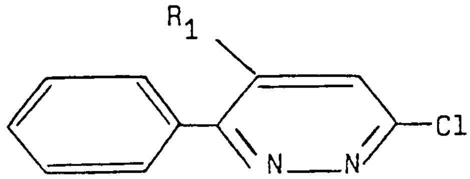

(2)

dans laquelle $R_1$ est tel que défini ci-dessus par chauffage entre 100 et 150 °C avec un large excès d'amine (1) éventuelement en présence de chlorure d'ammonium.

## Claims

**1.** Use of 5-alkylpyridazine derivatives of the formula

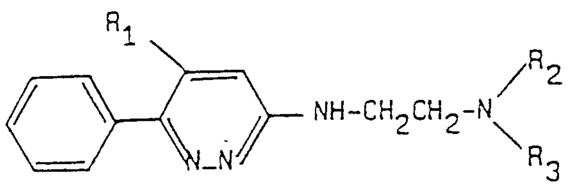

(II)

where $R_1$ is a $C_1$-$C_4$-alkyl group or a phenyl group; $R_2$ and $R_3$ independently are a $C_1$-$C_4$-alkyl group, or $R_2$ and $R_3$, taken with the nitrogen atom to which they are bonded, form a morpholino group, or of one of their pharmaceutically acceptable salts, for the preparation of pharmaceutical compositions for the treatment of degenerative syndromes associated with the senescence caused by a central cholinergic deficiency.

**2.** Use according to claim 1, characterized in that the pharmaceutical compositions are used for the treatment of senile dementia.

**3.** Use according to one of claims 1 or 2, characterized in that the pharmaceutical compositions are in the form of unit doses containing from 5 to 200 mg of the compound of formula (II) mixed with a pharmaceutical excipient.

**4.** 5-alkylpyridazine derivatives of the formula

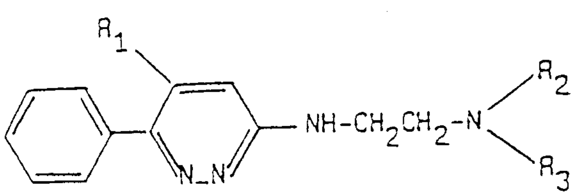

(II)

in which $R_1$ is a $C_2$-$C_4$-alkyl group;
$R_2$ and $R_3$ independently are a $C_1$-$C_4$-alkyl group, or $R_2$ and $R_3$, taken with the nitrogen atom to which they are bonded, form a morpholino group, or of one of their pharmaceutically acceptable salts.

**5.** Derivative according to claim 4, characterized in that $R_1$ is $-CH_2-CH_3$ or $-CH_2-CH_2-CH_3$.

**6.** Method for obtaining a 5-alkylpyridazine derivative according to one of claims 4 or 5, characterized in that it consists in reacting an amine of formula

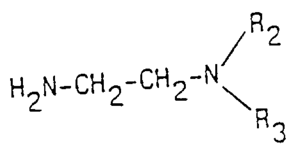

(1)

in which $R_2$ and $R_3$ are as defined above with a chlorine derivative of formula:

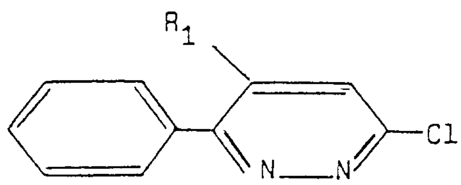

(2)

in which $R_1$ is as defined above by heating between 100 and 150 °C with a large excess of amine (1) if appropriate in the presence of ammonium chloride.

## Patentansprüche

**1.** Verwendung von 5-Alkylpyridazinderivaten der Formel

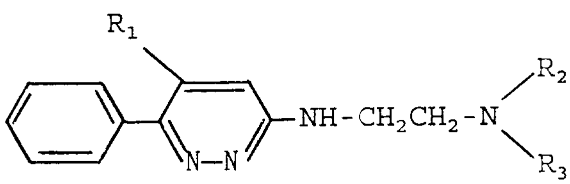

(II),

worin $R_1$ $C_{1-4}$-Alkyl oder Phenyl darstellt und
$R_2$ und $R_3$ unabhängig $C_{1-4}$-Alkyl bedeuten oder $R_2$ und $R_3$ zusammen mit dem Stickstoffatom, an das sie gebunden sind, eine Morpholinogruppe bilden,
oder eines ihrer pharmazeutisch akzeptablen Salze zur Herstellung pharmazeutischer Zusammensetzungen zur Behandlung von altersbedingten degenerativen Syndromen, die durch einen zentralen cholinergen Mangel hervorgerufen sind.

**2.** Verwendung nach Anspruch 1, dadurch gekennzeichnet, daß die pharmazeutischen Zusammensetzungen zur Behandlung der senilen Demenz verwendet werden.

**3.** Verwendung nach einem der Ansprüche 1 und 2, dadurch gekennzeichnet, daß die pharmazeutischen Zusammensetzungen in Form von Dosiereinheiten vorliegen, die 5 bis 200 mg der Verbindung der Formel (II) im Gemisch mit einem pharmazeutischen Excipiens enthalten.

**4.** 5-Alkylpyridazinderivate der Formel

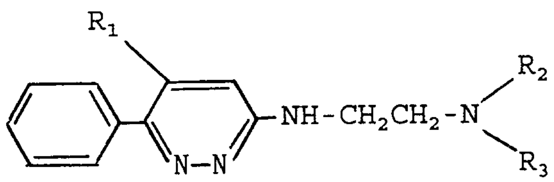

(II),

worin $R_1$ $C_{2-4}$-Alkyl darstellt und
$R_2$ und $R_3$ unabhängig $C_1$-$C_4$-Alkyl bedeuten oder
$R_2$ und $R_3$ zusammen mit dem Stickstoffatom, an das sie gebunden sind, eine Morpholinogruppe bilden,
oder eines ihrer pharmazeutisch akzeptablen Salze.

**5.** Derivate nach Anspruch 4, dadurch gekennzeichnet, daß $R_1$ $-CH_2-CH_3$ oder $-CH_2-CH_2-CH_3$ ist.

**6.** Verfahren zur Herstellung der 5-Alkylpyridazinderivate nach einem der Ansprüche 4 und 5, dadurch gekennzeichnet, daß ein Amin der Formel

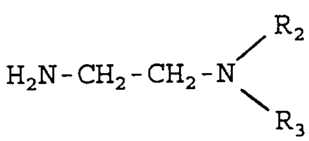

(1)

worin $R_2$ und $R_3$ dasselbe wie oben bedeuten, mit einem chlorierten Derivat der Formel

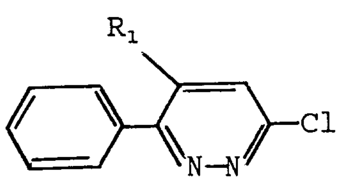

(2)

worin $R_1$ dasselbe wie oben bedeutet, durch Erhitzen auf 100 bis 150 °C unter Verwendung eines großen Überschusses an Amin (1), gegebenenfalls in Gegenwart von Ammoniumchlorid, zur Reaktion gebracht wird.